# FASCICULE DE BREVET EUROPEEN

(11) **EP 1 272 499 B1**
(45) Date de publication et mention de la délivrance du brevet: **09.03.2005**
(21) Numéro de dépôt: 01919561.9
(22) Date de dépôt: 26.03.2001
(51) Int. Cl.: C07H 15/04, C07H 5/06, C07H 11/00, C07H 7/033, C08B 37/00, A61K 31/70, A61P 29/00

(54) **COMPOSITIONS PHARMACEUTIQUES CONTENANT DES OLIGOSACCHARIDES ET LEUR PREPARATION**
PHARMAZEUTISCHE ZUSAMMENSETZUNGEN MIT OLIGOSACCHARIDEN UND DEREN HERSTELLUNG
PHARMACEUTICAL COMPOSITIONS CONTAINING OLIGOSACCHARIDES AND PREPARATION THEREOF

(30) Priorité: 28.03.2000 FR 0003910
(43) Date de publication de la demande: 08.01.2003
(73) Titulaire: Aventis Pharma S.A., 92160 Antony (FR)
(72) Inventeur: MOURIER, Pierre, 94220 Charenton Le Pont (FR); PERRIN, Elisabeth, 27000 Evreux (FR); VISKOV, Christian, 91130 Ris Orangis (FR)
(74) Mandataire: Rousseau, Pierrick Edouard
(86) Numéro de dépôt international: PCT/FR2001/000903
(87) Numéro de publication internationale: WO 2001/072762

(56) Documents cités:
- US-A- 5 449 688
- MCLEAN, MAITLAND W. ET AL: "Flavobacterium heparinum 2-O-sulfatase for 2-O-sulfato-.DELTA.4,5- glycuronate-terminated oligosaccharides from heparin" EUR. J. BIOCHEM. (1984), 145(3), 607-15, XP000974364
- LEE, GEORGE JIA-LONG ET AL: "Separation of reduced disaccharides derived from glycosaminoglycans by high-performance liquid chromatography" J. CHROMATOGR. (1981), 212(1), 65-73, XP000867181 cité dans la demande

## Description

La présente invention concerne les compositions pharmaceutiques contenant en tant que principe actif un oligosaccharide de formule : 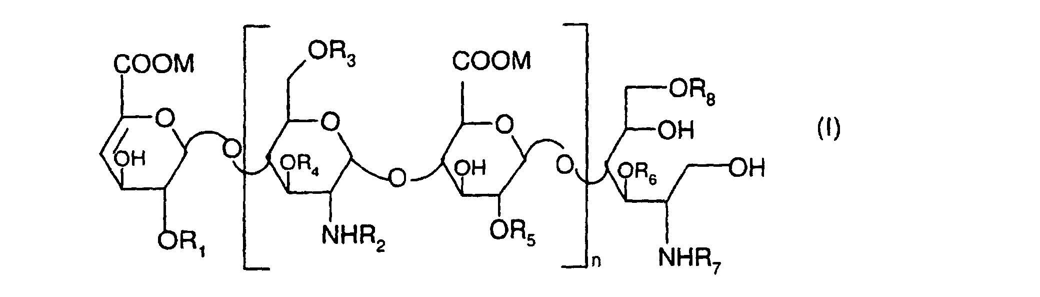 ou un mélange de ces oligosaccharides, les nouveaux oligosaccharides de formule (I), leurs mélanges et leurs procédés de préparation.

Dans la formule (I) n est un entier de 0 à 25, R₁, R₃, R₄, R₅, R₆ et R₈, identiques ou différents, représentent un atome d'hydrogène ou un radical SO₃M, R₂ et R. identiques ou différents, représentent un atome d'hydrogène ou un radical SO₃M ou COCH₃ et M est sodium, calcium, magnésium ou potassium, ou un mélange de ces oligosaccharides à l'exception de ceux pour lesquels n est égal à 0 et soit R₁, R₆ et R₈ sont des atomes d'hydrogène, R₇ représente un radical SO₃M ou COCH₃ et M est sodium, soit R₁ et R₆ représentent un atome d'hydrogène, R₇ représente un radical COCH₃, R₈ représente un radical SO₃M et M est sodium, soit R₆ représente un hydrogène, R₁, R₇ et R₈ représentent un radical SO₃M et M est sodium, soit R₆ et R₇ représentent des atomes d'hydrogène R₁ et R₈ représentent un radical SO₃M et M est sodium, soit R₁, R₆ et R₇ représentent un atome d'hydrogène, R₈ représente un radical SO₃M et M est sodium.

Ces oligosaccharides comportent ainsi un nombre pair de saccharides.

Dans la formule (I) R₄ et R₆ sont, de préférence, des atomes d'hydrogène.

Des oligosaccharides de formule (I) pour lesquels n est égal à 0, et soit R₁, R₆ et R₈ représentent un atome d'hydrogène, R₇ représente un radical SO₃M ou COCH₃ et M est sodium, soit R₁ et R₆ représentent un atome d'hydrogène, R₇ représente un radical COCH₃, R₈ représente un radical SO₃M et M est sodium, soit R₆ représente un atome d'hydrogène, R₁, R₇ et R₈ représentent un radical SO₃M et M est sodium ont déjà été décrits par G.H. LEE et coll., J. Chromat. 212, 65-73 (1981) mais aucune propriété pharmacologique n'est décrite pour ces produits.

Des oligosaccharides de formule (I) pour lesquels n est égal à 0 et soit R₆ et R₇ représentent des atomes d'hydrogène, R₁ et R₈ représentent un radical SO₃M et M est sodium, soit R₁, R₆ et R₇ représentent un atome d'hydrogène, R₈ représente un radical SO₃M et M est sodium sont décrits par MW McLEAN et coll., Eur. J. Biochem., 1984, 145, 607, sans aucune indication d'activité pharmacologique.

Les compositions pharmaceutiques préférées sont celles contenant un oligosaccharide de formule (I) pour lequel :
- n est un entier de 0 à 10 et, en particulier de 0 à 6 et encore plus particulièrement de 1 à 6.
- R₁, R₂, R₃, R₅, R₇, R₈ sont identiques ou différents et représentent un atome d'hydrogène ou un radical SO₃M et, en particulier R₁, R₂, R₃, R₅, R₇, R₈ sont des radicaux SO₃M,
- M est sodium.

Les compositions pharmaceutiques particulièrement préférées sont celles contenant un oligosaccharide de formule (I) pour lequel :
- n est égal à 0, R₁, R₇ et R₈ représentent un radical SO₃M, R₆ représente un atome hydrogène et M est sodium,
- n est égal à 1, R₁, R₂, R₃, R₅, R₇ et R₈, représentent un radical SO₃M, R₄ et R₆ représentent un atome d'hydrogène et M est sodium,
- n est égal à 2, R₁, R₂, R₃, R₅, R₇ et R₈, représentent un radical SO₃M, R₄ et R₆ représentent un atome d'hydrogène et M est sodium,
- n est égal à 3, R₁, R₂, R₃, R₅, R₇ et R₈, représentent un radical SO₃M, R₄ et R₆ représentent un atome d'hydrogène et M est sodium,
- n est égal à 4, R₁, R₂, R₃, R₅, R₇ et R₈, représentent un radical SO₃M, R₄ et R₆ représentent un atome d'hydrogène et M est sodium.

Les oligosaccharides de formule (I) à l'exception de ceux pour lesquels n est égal à 0 et soit R₁, R₆ et R₈ représentent un atome d'hydrogène, R₇ représente un radical SO₃M ou COCH₃ et M est sodium, soit R₁ et R₆ représentent un atome d'hydrogène, R₇ représente un radical COCH₃, R₈ représente un radical SO₃M et M est sodium, soit R₆ représente un hydrogène, R₁, R₇ et R₈ représentent un radical SO₃M et M est sodium. soit R₆, et R₇ représentent des atomes d'hydrogène, R₁ et R₈ représentent un radical SO₃M et M est sodium, soit R₁, R₆ et R₇ représentent un atome d'hydrogène, R₈ représente un radical SO₃M et M est sodium sont nouveaux et en tant que tels font partie de l'invention.

Les oligosaccharides de formule (I) peuvent être préparés par action d'un borohydrure de métal alcalin ou d'ammonium quaternaire sur des oligosaccharides de formule : 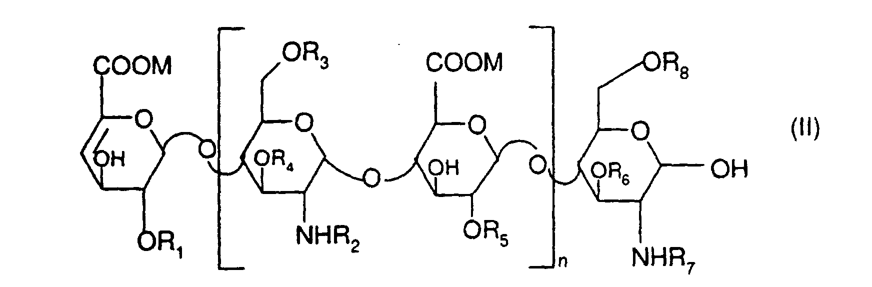 dans laquelle n est un entier de 0 à 25, R₁, R₃, R₄, R₅, R₆ et R₈, identiques ou différents, représentent un atome d'hydrogène ou un radical SO₃M, R₂ et R₇, identiques ou différents, représentent un atome d'hydrogène ou un radical SO₃M ou COCH₃ et M est sodium, calcium, magnésium ou potassium.

Cette réaction s'effectue en milieu aqueux, à une température voisine de 25°C, à un pH compris entre 7 et 10 et de préférence entre 9 et 10, pendant toute la durée de la réaction. Le maintien du pH est obtenu par addition d'une solution de soude à 0,5 mol/l. La réaction est arrêtée par acidification du milieu réactionnel, par exemple par addition d'acide acétique jusqu'à obtention d'un pH compris entre 4 et 5.

Comme borohydrure de métal alcalin, on peut citer le borohydrure de lithium, de sodium et de potassium.

Comme borohydrure d'ammonium quaternaire, on peut citer le borohydrure de tétrabutylammonium.

Les oligosaccharides de formule (II) peuvent être obtenus par séparation par chromatographie sur gel d'un mélange d'oligosaccharides (III) obtenu par dépolymérisation enzymatique de l'héparine ou dépolymérisation basique de l'ester benzylique de l'héparine ou d'un ester benzylique d'héparine d'hémisynthèse.

Cette chromatographie s'effectue sur des colonnes remplies de gel de type polyacrylamide-agarose tel que celui commercialisé sous la marque Ultrogel ACA202^{R} (Biosepra). De préférence, on utilise une batterie de colonnes de gel polyacrylamide agarose. Le nombre de colonnes utilisées est adapté en fonction du volume, du gel et des oligosaccharides à séparer. Le mélange est élué par une solution contenant un tampon phosphate et du chlorure de sodium. De préférence, la solution tampon phosphate est une solution à 0,02 mol/l de NaH₂PO₄/Na₂HPO₄ (pH 7) contenant 0,1 mol/l de chlorure de sodium. La détection des différentes fractions est réalisée par spectrométrie UV (254 nm) et ionique (IBF). Les fractions ainsi obtenues peuvent ensuite être éventuellement purifiées par exemple par chromatographie SAX (strong anion exchange) selon les méthodes connues de l'homme du métier et notamment selon les méthodes décrites par K.G. Rice et R.J Linhardt, Carbohydrate Research 190, 219-233 (1989), A. Lamkjaer, S.H. Hansen et P.B. Ostergaard, Carbohydrate Research, 266, 37-52 (1995) et dans le brevet WO90/01501 (exemple 2). Les fractions sont ensuite lyophilisées puis désalées sur une colonne remplie de gel telle qu'une colonne de gel Séphadex G10^{R} (Pharmacia Biochemicals).

Lorsque la purification n'est pas réalisée par chromatographie SAX, les lyophilisats peuvent être éventuellement purifiés par précipitation simple ou fractionnée selon les méthodes connues de l'homme du métier et notamment selon la méthode décrite dans le brevet FR2548672. De façon générale, on opère selon le protocole suivant :

La fraction lyophilisée à purifier est dissoute à 25°C dans environ dix volumes d'eau distillée. Par ajout de méthanol ou d'éthanol, on fait précipiter l'oligosaccharide désiré en contrôlant son enrichissement par chromatographie CLHP (Chromatographie Liquide Haute Performance). L'ajout de méthanol ou d'éthanol est déterminé en fonction de la pureté et du rendement désiré du dit oligosaccharide. De même, cette opération peut être réalisée en plusieurs étapes successives à partir de la solution initiale de lyophilisat. Pour cela, on rajoute l'agent insolubilisant (méthanol ou éthanol) par petites portions et on isole après chaque ajout le précipité obtenu. Les précipités ainsi préparés sont analysés par chromatographie CLHP. Selon la pureté et le rendement désiré, on rassemble les fractions adéquates de précipité.

Selon une variante de la présente invention, la fraction lyophilisée à purifier peut être dissoute dans 10 à 200 volumes d'eau contenant de 0 à 30% d'acétate de sodium. Le pourcentage d'acétate de sodium sera ajusté au préalable en fonction de la nature de l'oligosaccharide à traiter (fonction de la taille). Par ajout de méthanol, on fait précipiter l'oligosaccharide désiré en contrôlant son enrichissement par chromatographie CLHP (Chromatographie Liquide Haute Performance). L'ajout de méthanol est déterminé en fonction de la pureté et du rendement désiré du dit oligosaccharide. De même, cette opération peut être réalisée en plusieurs étapes successives à partir de la solution initiale de lyophilisat. Pour cela, on rajoute l'agent insolubilisant (méthanol) par petites portions et on isole après chaque ajout le précipité obtenu. Les précipités ainsi préparés sont analysés par chromatographie CLHP. Selon la pureté et le rendement désiré, on rassemble les fractions adéquates de précipité.

Pour les intermédiaires de formule (U) pour lesquels n = 0, 1 ou 2, il est préférable de partir d'un mélange (III) obtenu par dépolymérisation enzymatique.

Cette dépolymérisation s'effectue au moyen d'héparinase I (EC 4.2.2.7), au sein d'une solution tampon phosphate de pH7, en présence de chlorure de sodium et de BSA (Albumine de Sérum Bovin), à une température comprise entre 10 et 18°C et, de préférence 15°C, pendant 8 à 10 jours et, de préférence, 9 jours. La dépolymérisation est arrêtée par exemple par chauffage du milieu réactionnel à 100°C pendant 2 minutes et le mélange est récupéré par lyophilisation. Il est préférable d'utiliser 7 UI d'héparinase I pour 25 g d'héparine. La solution tampon phosphate comprend généralement 0,05 mol/l de NaH₂PO₄/Na₂HPO₄ (pH 7) en présence de 0,1 mol/l de chlorure de sodium. La concentration en BSA est généralement de 2%.

Pour les intermédiaires de formule (II) pour lesquels n = 0, 1, 2, 3 ou 4, il est préférable de partir d'un mélange (III) obtenu par dépolymérisation d'un ester benzylique de l'héparine.

L'ester benzylique de l'héparine peut être préparé selon les méthodes décrites dans les brevets US5389618, EP40144, FR2548672. Le taux d'estérification sera de préférence compris entre 50 et 100 %. De façon préférentielle, il sera compris entre 70 et 90%.

La dépolymérisation s'effectue en milieu aqueux, au moyen d'un hydroxyde de métal alcalin (hydroxyde de lithium, soude, potasse ou hydroxyde de césium par exemple) ou d'un hydroxyde d'ammonium quaternaire (hydroxyde de tétrabutylammonium par exemple), de préférence, à une molarité comprise entre 0,1 et 0,2 mol/l, à une température comprise entre 40 et 80°C, pendant 5 à 120 minutes. Dans un mode préféré, on opère pendant 5 à 15 minutes, à une température comprise entre 60 et 70°C, avec une solution d'hydroxyde de sodium 0,15 mol/l. La réaction de dépolymérisation est arrêtée par neutralisation par addition d'un acide tel que l'acide acétique. Après addition de 10% en poids par volume d'acétate de sodium, le mélange d'oligosaccharides est précipité par addition de méthanol, de préférence, 2 volumes pour 1 volume de milieu réactionnel et filtré.

Selon un aspect préféré de l'invention, le mélange d'oligosaccharides obtenu après dépolymérisation chimique, sous forme d'une solution aqueuse, est enrichi par ultrafiltration sur membranes avec un seuil de coupure nominal approprié (type Pellicon en cellulose régénérée commercialisées par Millipore); le type de membrane étant adapté en fonction du type d'oligosaccharides enrichis à récupérer. Pour les oligosaccharides (II) pour lesquels n = 0, on utilisera une membrane de seuil de coupure nominal de 1 kDa, pour les oligosaccharides (II) pour lesquels n = 1, on utilisera une membrane 1 kDa ou 3 kDa, pour les oligosaccharides (II) pour lesquels n = 2, on utilisera une membrane 3 kDa, pour les oligosaccharides (II) pour lesquels n = 3 ou 4, on utilisera une membrane 5 kDa. Au cours de cette opération, le perméat est récupéré et le rétentat rejeté. Ainsi, la fraction de produit enrichi peut représenter de 50 à 10% du mélange d'oligosaccharides initial tout en conservant au moins 80% de l'oligosaccharide désiré.

Pour les intermédiaires de formule (II) pour lesquels n = 0 à 25, il est préférable de partir d'un mélange (III) obtenu par dépolymérisation d'un ester benzylique de polysaccharide sulfaté d'hémisynthèse. L'ester benzylique de polysaccharide sulfaté d'hémisynthèse est préparé à partir de polysaccharides sulfatés d'hémisynthèse obtenus à partir du polysaccharide K5 et selon les méthodes décrites dans les brevets WO94/29352 et WO96/14425. Les conditions d'estérification, de dépolymérisation et de récupération sont les mêmes que celles décrites précédemment pour l'ester benzylique d'héparine.

Dans tous les procédés précédents, l'héparine initiale peut être d'origine porcine, ovine, caprine ou bovine et peut provenir des mucus, poumons ou peaux des animaux.

De préférence, on utilise une héparine de mucus porcin, ovin ou de poumons de boeuf et encore plus préférentiellement de mucus porcin ou de poumon de boeuf.

Les oligosaccharides de formule (I) présentent des propriétés anti-inflammatoires et peuvent ainsi être utilisées pour la prévention ou le traitement de maladies liées à un processus inflammatoire impliquant la production de substances cytotoxiques telle que le monoxyde d'azote (NO) dont la forme inductible est libérée notamment par les neutrophiles ou les macrophages lorsque ceux-ci migrent et sont activés au niveau d'un tissu. La migration, l'activation et l'adhésion des neutrophiles se produit au niveau des zones tissulaires ischémiées à la suite d'une occlusion ou d'un spasme d'une artère vascularisant ce tissu. Ces ischémies peuvent se produire soit au niveau cérébral (accident vasculaire cérébral), soit au niveau du myocarde (infarctus du myocarde), soit au niveau des membres inférieurs (ischémies dites périphériques). Les oligosaccharides de formule (I) peuvent ainsi être utilisées pour la prévention et/ou le traitement des maladies neurodégénératives pour lesquelles l'inflammation cérébrale joue un rôle délétère pouvant conduire à la mort parmi lesquelles on peut citer les ischémies cérébrales, les ischémies cardiaques (infarctus du myocarde), les ischémies périphériques, les traumatismes du système nerveux central et notamment les traumatismes crâniens, spinaux et cranio-spinaux, la sclérose en plaques, les douleurs neuropathiques et les neuropathies périphériques, les maladies du motoneurone dont la sclérose latérale amyotrophique, l'atrophie spinale progressive, l'atrophie musculaire infantile et la sclérose latérale primaire, le neuro-sida, la maladie d'Alzheimer, la maladie de Parkinson et la Chorée de Huntington et certaines formes d'ostéoarthrites notamment à localisation articulaire.

L'activité anti-inflammatoire de ces produits est démontrée in vivo dans le test de production de NOx (nitrite et nitrate) induite par un lipopolysaccharide (LPS) provenant d'E. Coli selon le protocole décrit par M. YAMASHITA et coll., Eur. J. Pharmacol, 338, 2, 151-158 (1997) ou J.E. SHELLITO et coll. Am. J. Respir. Cell Mol. Biol., 13, 1, 45-53 (1995).

On injecte, à des souris CDI mâles (Charles River, 25-35g), à T0 par voie intraveineuse bolus 0,5 mg/kg de l'oiigosaccharide, à T+15 minutes, par voie sous-cutanée 1 ou 2 mg/kg de l'oligosaccharide. A T+30 minutes, on administre 100 mg/kg de lipopolysaccharide (LPS) provenant d'E. Coli (Sigma L3129, sérotype 0127:B8). A T+3 heures on injecte à nouveau par voie sous-cutanée 1 ou 2 mg/kg de l'oligosaccharide. A T+5 heures 30 minutes, un échantillon de sang est récupéré par ponction à l'oeil et les concentrations en NOx (nitrite et nitrate) dans le plasma sont déterminées avec la méthode colorimétrique de Griess après réduction du nitrate en nitrite par nitrate réductase de la manière suivante : 12 ml de l'échantillon de plasma sont mélangés avec 88 ml d'eau déionisée et incubés dans le noir 1 heure à température ambiante avec 40 ml de tampon phosphate (0,31 M, pH 7,5). 20 ml de β-NADPH (nicotinamide adénine dinucléotide phosphate réduit) (0,86mM), 20 ml de FDA (flavine adénine dinucléotide) (0,11 mM) et 20 ml de nitrate réductase (2U/ml) (Boehringer Mannheim). 10 ml de ZnSO₄ (1M) sont ajoutés pour précipiter les protéines et après mélange, les échantillons sont centrifugés à 20 000g pendant 5 minutes. Finalement, 50 ml du supemageant sont incubés 10 minutes à température ambiante avec 100 ml du réactif de Griess (sulfanilamide à 1 % dans un mélange acide phosphorique/naphtyéthylènediamine 0,1% dans l'eau déionisée (V/V)). Les densités optiques sont lues à 540 nm avec un spectrophotomètre microplaques; chaque point étant déterminé 2 fois. KNO₃ et NaNO₂ sont utilisés comme standard pour la méthode colorimétrique.

Dans ce test, les oligosaccharides selon l'invention inhibent à plus de 50 % la formation de NOx.

Par ailleurs, les oligosaccharides de formule (I) augmentent la survie et la croissance des motoneurones et sont donc particulièrement utiles dans la prévention et/ou le traitement des maladies motoneuronales telles que la sclérose latérale amyotrophique, l'atrophie spinale progressive, l'atrophie musculaire infantile, la sclérose latérale primaire.

Il est connu que les cultures de motoneurones meurent par apoptose si elles sont effectuées en absence de support trophique (BDNF, NT5 par exemple). Il a maintenant été trouvé que les oligosaccharides selon l'invention permettent la survie et la croissance des motoneurones. Cette activité a été testée sur des co-cultures d'astrocytes et de motoneurones privées de facteur neurotrophique selon le protocole suivant :

### CULTURES ENRICHIES EN MOTONEURONES :

Les cultures enrichies en motoneurones sont préparées en utilisant la méthode de centrifugation décrite par R.L. SCHNAAR et A.E. SCHAFFNER, J. Neurosci., 1, 204-217 (1981) et modifiée par W. CAMU et C.E. HENDERSON. J. Neurosci. Methods, 44, 59-70 (1992). Des moelles épinières d'embryons de rat E15 sont disséquées stérilement et débarassées des cordes spinales dorsales. Elles sont ensuite coupées et incubées 15 minutes à 37°C dans du PBS (phosphate buffer saline : NaCl 137 mM, Kcl 2,68 mM, Na₂HPO₄ 6,45 mM, KH₂PO₄ 1,47 mM) auquel on a ajouté 0.05% de trypsine. La dissociation des cellules est complétée par trituration avec l'extrémité d'une pipette de 1 ml dans le milieu de culture additionné de 0,1% d'albumine de sérum bovin (BSA) et de 0,1 mg/ml de ADNase. La suspension de cellules est étalée sur une bande de métrizamide 6,5% poids/volume dans du milieu L15 (commercialisé par Gibco BRL) et centrifugée à 500 g pendant 15 minutes. La bande de l'interface contenant les motoneurones est récupérée, diluée dans du milieu L15 et incubée 45 minutes à température ambiante dans des boites de culture préalablement enduites d'anti-IgG de souris et de supernageant hybridome MC192 (Chandler CE et coll., J. Biol. Chem., 259, 6882 (1984). Les cellules suspendues sont lavées avec du milieu L15 et les motoneurones sont élués sous faible agitation avec du supemageant hybridome MC192. Les motoneurones sont étalés à une densité de 650 cellules pour 24 mm dans des boites de culture sur les monocouches d'astrocytes dans du milieu L15 auquel on a ajouté du bicarbonate de sodium (22mM), de la coalbumine (0,1 mg/ml), de la putrescine (0,1 mM), de l'insuline (5 µg/ml), du sélénite de sodium (31 nM), du glucose (20 mM), de la progestérone (21 nM), de la pénicilline (100 UI/ml) et de la streptomycine (100 ug/ml). Les cultures sont maintenues à 37°C dans une atmosphère humidifiée à 5% de CO₂.

### CULTURE D'ASTROCYTES DE MOELLE EPINIERE:

Les astrocytes sont obtenus à partir d'embryons de rais selon la méthode de R.P. SANETO ET J. DE VELLIS, in Neurochemistry, a practical approach (A.J. TURNER et H.S. St JOHN) IRL Press, Oxford-Washington DC, p27-63 (1987) légèrement modifiée. Les moelles épinières sont disséquées stérilement, débarassées des méninges et des ganglions dorsaux. Cinq à dix moelles épinières sont transférées dans du PBS (phosphate buffer saline : NaCl 137 mM, Kcl 2,68 mM, Na₂HPO₄ 6,45 mM, KH₂PO₄ 1,47 mM) et coupées avant incubation à 37°C pendant 25 minutes dans du PBS auquel on a ajouté 0,25% de trypsine. Le traitement enzymatique est stoppé par addition de 10 ml de milieu Dubelcco-Eagle modifié (DMEM) auquel on a ajouté 10% de sérum foetal de veau (FCS) et les cellules sont collectées par centrifugation. Une autre étape de dissociation mécanique est effectuée en utilisant l'extrémité d'une pipette de 1 ml. Les cellules sont étalées à une densité de 1,5x10⁶ cellules pour 25 cm² de milieu de culture dans du DMEM à 10% de FCS. Après 2 jours in vitro les cultures sont alimentées chaque jour tout au long de la durée de l'étude. Quand une monocouche visible de cellules est obtenue, les cultures sont agitées 48 heures à 250 rpm et, le lendemain, les monocouches sont traitées par du cytosine arabinoside (10⁻⁵ M) pendant 48 heures. Les monocouches d'astrocytes sont ensuite amplifiées à une densité de cinq pour 35 mm sur des plaques de culture pour des flacons de culture de 25 cm² au début de l'étude.

Les cultures d'astrocytes spinaux sont composées à plus de 98% de cellules immunoréactives pour la protéine gliale fibrilaire acide (GFAP).

Les monocouches d'astrocytes sont exposées soit au PBS seul (témoins) soit au produit à tester en solution dans du PBS pendant 24 heures à la concentration de 0,1 ng/ml à 10 ng/ml. Les monocouches d'astrocytes sont ensuites lavées avec du DMEM et maintenues 2 heures avec du milieu de culture où les motoneurones sont ajoutés. Deux heures après l'alimentation et durant 2 ou 3 jours, le véhicule ou le produit à tester est encore ajouté au milieu de culture.

### IDENTIFICATION IMMUNOCHIMIQUE DES MOTONEURONES

Les cellules sont fixées dans 4% de paraformaldéhyde et 0,1 % de glutaraldéhyde dans du PBS (pH 7,4 à 4°C pendant 15 minutes). Les cultures sont ensuite lavées et les sites non spécifiques bloqués avec 2 % d'albumine de sérum bovin (BSA) dans du PBS et 0,1 % de Triton X100^{R}. Ces cultures sont successivement incubées avec des anticorps de p75^{LNGRF} (article de Chandler précédemment cité), une nuit à 4°C et avec du sérum de chèvre biotinylisé 1/125 Gibco) et du streptavidenperoxydase (1/200, Gibco) pendant 60 minutes. Les anticorps sont visualisés en utilisant la réaction DAB/peroxyde d'hydrogène.

### COMPTAGE DES CELLULES ET ANALYSE STATISTIQUE

Les cellules immunoréactives pour le récepteur neutrophine de faible activité p75^{Ingfr} et exhibant des neurites plus longs que les diamètres de 4 cellules sont considérés comme des motoneurones viables. Le nombre de motoneurones est évalué par comptage des cellules marquées dans une surface de 0,825 cm² sous microscope grossissant 200 fois. Les valeurs sont exprimées comme un nombre de motoneurones par cm² ou un pourcentage du nombre de motoneurones présents dans les cultures maintenues en l'absence de facteur trophique comparé au contrôle. Les expériences sont réalisées au moins 3 fois.

Les analyses statistiques sont effectuées en utilisant le test de Student (t-test).

Par prétraitements avec les oligosaccharides de la présente invention, le nombre de motoneurones qui poussent sur la monocouche d'astrocytes est augmenté de 20 à 50%.

Les exemples suivants sont représentatifs de la préparation des oligosaccharides de formule (I) et des intermédiaires.

Dans ces exemples les significations des abréviations sont les suivantes :
ΔIs : (acide 4-déoxy-2-*O*-sulfo-α-L-thréo-hex-enopyranosyluronique)-(1→4)-2-déoxy-2-sulfoamino-6-*O*-sulfo- α -D-glucopyranose, sel de tétrasodium ou bien ΔUA*p*2S- (1→4)-a-D-GlcN*p*2S6S
Is : (acide 2-sulfo- α-L-idopyranosyluronique)-(1→4)-2-déoxy-2-sulfoamino-6-*O*-sulfo- α-D-glucopyranose, sel de tétrasodium (acide 2-sulfo- α -L-idopyranosyluronique)-(1→4)-2-déoxy-6-*O*-sulfo-2-sulfoamino- α-D-glucopyranose, sel de tétrasodium ou bien a α -L-IdoA*p*2S- (1→4)- α -D-GlcN*p*2S6S
IIs : (acide α-L-idopyranosyluronique)-(1→4)-2-déoxy-6-*O*-sulfo-2-sulfoamino - α-D-glucopyranose, sel de trisodium ou bien α-L-IdoA*p*- (1→4)- α-D-GlcN*p*2S6S
IIIs : (acide 2-sulfo- α-L-idopyranosyluronique)-(1→4)-2-déoxy-2-sulfoamino- α-D-glucopyranose, sel de trisodium ou bien α-L-IdoA*p*2S- (1→4)- α-D-GlcN*p*2S
IdoA*p* : acide idopyranosyluronique
GlcN*p* : 2-amino-2-déoxy-glucopyranose
ΔUa*p* : acide 4-déoxy- α-L-thréo-hex-enopyranosyluronique
S : sulfate.

### EXEMPLES DE PREPARATION DES MELANGES INTERMEDIAIRES DE FORMULE (II)

### EXEMPLE A - préparation des oligosaccharides de formule (II) pour lesquels n = 0, 1 et 2 par dépolymérisation enzymatique et séparation

Dissoudre 25 g d'héparine dans 250 ml d'une solution tampon phosphate contenant 0,05 mol/l NaH₂PO₄/Na₂HPO₄ (pH = 7), 0,2 mol/l de chlorure de sodium et 2 % de BSA (Albumine de Sérum Bovin). On introduit dans le mélange 7 UI d'héparinase I (EC 4.2.2.2.7) et la solution obtenue est refroidie à 15°C puis maintenue à cette température pendant toute la durée de la réaction de dépolymérisation. L'avancement de la réaction est suivie par des prélèvements échelonnés d'aliquotes et analysés par chromatographie sur perméation de gel. Au bout de 9 jours, la réaction enzymatique est arrêtée en chauffant le milieu réactionnel à 100°C pendant deux minutes. Le mélange refroidi est ensuite lyophilisé. On obtient ainsi un mélange d'oligosaccharides (III).

Le mélange d'oligosaccharides (III) obtenu est ensuite chromatographie selon la méthode suivante : La chromatographie s'effectue sur des colonnes remplies avec du gel polyacrylamide-agarose connu sous la dénomination Ultrogel ACA 202 et le mélange est élué par une solution contenant un tampon phosphate (0,02 mol/l NaH₂PO₄/Na₂HPO₄) pH = 7 et 0,1 mol/l de chlorure de sodium. La détection est réalisée en spectrométrie UV (254 nm) et ionique (IBF). Les produits peuvent être éventuellement purifiés par chromatographie SAX (strong anion exchange) ou par précipitation fractionnée selon la méthode décrite dans le brevet FR 2548672. Les fractions de produit récupéré sont lyophilisées puis désalées sur une colonne remplie de gel sephadex G10^{R} (Pharmacia Biochemicals).

Par cette méthode, on obtient 3 g de disaccharide ΔIs, 1100 mg d'un mélange d'hexasaccharide contenant typiquement 55 % de dérivé ΔIs-Is-Is , 35 % de ΔIs-Is-IIs et 10 % de ΔIs-Is-IIIs. Ce dernier mélange peut être purifié selon les méthodes connues de l'homme du métier pour en séparer chacun des constituants ou utilisé tel quel pour la transformation en dérivés réduits de formule (I).

### EXEMPLE B - préparation des oligosaccharides de formule (II) pour lesquels n=0,1, 2, 3 ou 4 par dépolymérisation de l'ester benzylique d'héparine et séparation

### a - Préparation de l'ester benzylique de l'héparine

L'ester benzylique de l'héparine est préparé selon l'exemple 3 du brevet US 5,389,618.

### b- Dépolymérisation

Dissoudre 100 g d'ester benzylique de l'héparine dans 1,9 l d'eau déminéralisée. Le mélange est porté à 60°C sous agitation. Après l'obtention d'une solution homogène, on introduit en une seule fois environ 35 ml d'une solution d'hydroxyde de sodium à 23 %. Après 10 minutes de réaction, la solution est ensuite refroidie et neutralisée par 80 ml d'une solution d'acide acétique environ 2 N. A cette solution, on ajoute 10 % en poids/volume d'acétate de sodium. Le mélange d'oligosaccharides est précipité par addition d'environ 2 volumes de méthanol. Le précipité est isolé par filtration, lavé au méthanol à deux reprises puis séché sous pression réduite à 50°C. Après séchage, on obtient 73,8 g d'un mélange d'oligosaccharides (II).

### c- Enrichissement en oligosaccharide pour lequel n = 1

Dissoudre 30 g du mélange d'oligosaccharides obtenu précédemment dans environ 35 volumes d'eau. Cette solution est ultrafiltrée sur membrane 3 kDa (Pellicon). Lorsque 600 ml de perméat on été soutirés, on dilue le rétentat par 500 ml d'eau. L'opération est poursuivie jusqu'à soutirement de 450 ml de perméat supplémentaires. Les deux fractions de perméat sont réunies puis concentrées à sec sous pression réduite. On obtient 6,1 g d'un solide blanc jaunâtre. L'analyse du solide par chromatographie de perméation sur gel indique qu'il contient environ 30 % d'oligosaccharide de formule (II) pour lequel n=1.

### d - Fractionnement des mélanges d'oligosaccharides ultrafiltrés

Le mélange enrichi est fractionné sur des colonnes remplies avec du gel polyacrylamide-agarose connu sous la dénomination Ultrogel ACA 202 (on utilise 4 colonnes en série d'un diamètre de 10 cm et d'une hauteur de 50 cm). 5 g du mélange enrichi par ultrafiltration sont dissous dans 25 ml d'eau puis élués par une solution 0,2 mol/l de chlorure de sodium à la vitesse de 5 ml/min. On recueille en bas de colonne des fractions de 25 ml. La détection des produits est réalisée en spectrométrie UV (254 nm) et ionique (IBF). Les fractions de produit pour lequel n = 1 sont récupérées, lyophilisées puis désalées sur une colonne remplie de gel Sephadex G10. Après lyophilisation, on obtient 1 g de tétrasaccharide contenant typiquement 70 % de dérivé ΔIs-Is de formule II (R₁, R₂, R₃, R₅, R₇, R₈ = SO₃Na; R₄, R₆ = H et M = Na). Le dérivé ΔIs-Is peut être éventuellement purifié par chromatographie SAX (strong anion exchange) ou selon un aspect préférentiel par précipitation fractionnée selon la méthode décrite dans le brevet FR 2548672 et la variante décrite dans la présente invention.

### EXEMPLES DE PREPARATION DES OLIGOSACCHARIDES DE FORMULE (I)

### EXEMPLE 1

Dans un réacteur on introduit une solution à 25°C de 300 mg d'un oligosaccharide de formule (II) dans laquelle n est égal à 0, R₁, R₇ et R₈ représentent un radical SO₃M, R₆ représente un atome d'hydrogène et M est sodium, dans 2 ml d'eau. Sous agitation, on ajoute en une fois 212 mg de borohydrure de sodium. Le pH est alors ajusté entre 9 et 10 par addition d'une solution d'hydroxyde de sodium à 0,5 mol/l. Après 12 heures, on ajoute de façon progressive de l'acide acétique jusqu'à obtention d'un pH compris entre 4 et 5. Le mélange est agité 1 heure puis on réajuste le pH à 6,7 par addition de soude à 0,5 mol/l. Le mélange est alors concentré à sec à 50°C sous pression réduite. Le concentrât est dispersé sous agitation magnétique dans 10 ml de méthanol. Après une nuit de sédimentation, la suspension est filtrée sur membrane Whatman GF/B. Le solide sur filtre est dissous par passage de 2 portions de 10 ml d'eau distillée. Cette solution est alors concentrée à sec à 50°C sous pression réduite. On obtient 580 mg d'un solide blanc. Le solide est ensuite dispersé sous agitation magnétique dans 15 ml de méthanol. Après 30 minutes d'agitation, la suspension est filtrée sur membrane Whatman GF/B. Le gâteau est dissous par passage de 2 portions de 10 ml d'eau distillée. La solution obtenue est concentrée à sec à 50°C sous pression réduite. On obtient ainsi 250 mg d'un oligosaccharide de formule (I) pour lequel n est égal à 0, R₁, R₇ et R₈ représentent un radical SO₃M, R₆ représente un atome d'hydrogène et M est sodium, sous la forme d'un mélange des diastéréoisomères. On note de I à II les sucres constitutifs des disaccharides, I étant le résidu réduit et II le résidu acide uronique insaturé [(acide 4-déoxy-2-*O*-sulfo-α-L-thréo-hex-4-enopyranosyluronique-(1→4)-2-déoxy-6-*O*-sulfo-2-sulfoamino-D-glucitol, sel de tétrasodium); (acide 4-déoxy-2-*O*-sulfo- α-L-thréo-hex-4-enopyranosyluronique-(1→4)-2-déoxy-6-*O*-sulfo-2-sulfoamino-D-manitol, sel de tétrasodium) : Spectre proton dans D₂O, 600MHz, T=305K, δ en ppm: 3,38 (1H, m, H-2^{(I)}), 3,70 et 3,75 (1H chacun, respectivement dd, J=6 et 12Hz et dd, J=5 et 12Hz, 2H- 1^{(I)}), 3,86 (1H, t, J=5Hz, H-3^{(I)}), 4,13 (1H, dd, J=8 et 11Hz, H-6^{(I)}), 4,18 (1H, m, H-4^{(I)}), 4,25 (2H, m, H-6^{(I)} et H-3^{(II)}), 4,35 (1H, m, H-5^{(I)}), 4,65 (1H, m, H-2^{(II)}), 5,67 (1H, s, H-1^{(II)}), 6,00 (1H, d, J=5Hz, H-4^{(II)})].

### EXEMPLE 2

Dans un réacteur on introduit une solution à 25°C de 60 mg d'un oligosaccharide de formule (II) dans laquelle n est égal à 1, R₁, R₂, R₃, R₅, R₇ et R₈ représentent un radical SO₃M, R₄ et R₆ représentent un atome d'hydrogène et M est sodium, dans 1,2 ml d'eau. Sous agitation, on ajoute en une fois 18 mg de borohydrure de sodium. Le pH est alors ajusté entre 9 et 10 par addition d'une solution d'hydroxyde de sodium à 0,5 mol/l. Après 12 heures, on ajoute de façon progressive de l'acide acétique jusqu'à obtention d'un pH compris entre 4 et 5. Le mélange est agité 1 heure puis on ajoute 5 ml de méthanol. La suspension est filtrée sur membrane Whatman GF/B et le solide récupéré est rincé par 2 fois 0,5 ml de méthanol. Après séchage, on obtient 42 mg d'un oligosaccharide de formule (I) pour lequel n est égal à 1, R₁, R₂, R₃, R₅, R₇ et R₈ représentent un radical SO₃M, R₄ et R₆ représentent un atome d'hydrogène et M est sodium, sous forme d'un mélange des diastéréoisomères. On note de I à IV les sucres constitutifs des tétrasaccharides, I étant le résidu réduit et IV le résidu acide uronique insaturé [(acide 4-déoxy-2-*O*-sulfo- α-L-thréo-hex-4-enopyranosyluronique-(1→4)-2-déoxy-6-*O*-sulfo-2-sulfoamino- α-D-glucopyranosyl-(1→4)-acide 2-*O*-sulfo- α-L-idopyranosyluronique -(1 →4)-2-déoxy-2-sulfoamino-6-*O*-sulfo-D-glucitol, sel d'octasodium) ; (acide 4-déoxy-2-*O*-sulfo- α-L-thréo-hex-4-enopyranosyluronique-(1→4)-2-déoxy-6-*O*-sulfo-2-sulfoamino- α-D-glucopyranosyl-(1→4)-acide 2-*O*-sulfo- α-L-idopyranosyluronique -(1→4)-2-déoxy-6-*O*-sulfo-2-sulfoamino-D-manitol, sel d'octasodium) : Spectre proton dans D₂O, 400MHz, T=298K, δ en ppm: 3,25 (1H, dd, J=10 et 3Hz, H-2^{(III)}), 3,37 (1H, m, H-2^{(I)}), 3,59 (1H, m, H-3^{(III)}), 3,75 (2H, m, 2H-1^{(I)}), 3,79 (1H, t, J=9Hz, H-4^{(III)}), 3,86 (1H, m, H-3^{(I)}), entre 4,05 et 4,40 (10H, massif, H-4^{(I)}/H-5^{(I)}/2H-6^{(I)}, H-2^{(II)}/H-3^{(II)}/H-4^{(II)}), 2H-6^{(III)}, H-3^{(IV)}), 4,58 (1H, m, H-2^{(IV)}), 4,60 (1H, m, H-5^{(II)}), 5,27 (1H, d, J=4Hz, H-1^{(II)}), 5,42 (1H, d, J=4Hz, H-1^{(III)}), 5,47 (1H, d, J=2Hz, H-1^{(IV)}), 5,95 (1H, d, J=5Hz, H-4^{(IV)})].

### EXEMPLE 3

Dans un réacteur on introduit une solution à 25°C de 100 mg d'un oligosaccharide de formule (II) dans laquelle n est égal à 2, R₁, R₂, R₃, R₅, R₇ et R₈ représentent un radical SO₃M, R₄ et R₆ représentent un atome d'hydrogène et M est sodium, dans 2 ml d'eau. Sous agitation, on ajoute en une fois 20 mg de borohydrure de sodium. Le pH est alors ajusté entre 9 et 10 par addition d'une solution d'hydroxyde de sodium à 0,5 mol/l. Après 12 heures, on ajoute de façon progressive de l'acide acétique jusqu'à obtention d'un pH compris entre 4 et 5. Le mélange est agité 1 heure puis on réajuste le pH à 6,7 par addition de soude à 0,5 mol/l. Le mélange est alors dilué avec de l'eau distillée en quantité suffisante pour obtenir 20 ml. On ajoute 2,5 g d'acétate de sodium et on coule 3 volumes de méthanol. La suspension est filtrée sur membrane Whatman GF/B et le solide récupéré est rincé par 2 fois 2 ml de méthanol. Après séchage, on obtient 61 mg d'un oligosaccharide de formule (I) pour lequel n est égal à 2, R₁, R₂, R₃, R₅, R₇ et R₈ représentent un radical SO₃M, R₄ et R₆ représentent un atome d'hydrogène et M est sodium, sous forme d'un mélange des diastéréoisomères. On note de 1 à VI les sucres constitutifs des hexasaccharides, I étant le résidu réduit et VI le résidu acide uronique insaturé. [(acide 4-déoxy-2-*O*-sulfo- α-L-thréo-hex-4-enopyranosyluronique -( 1→4)- 2-déoxy-2-sulfoamino-6-*O*-sulfo- α-D-glucopyranosyl-(1→4)- acide 2-*O*-sulfo- α-L-idopyranosyluronique -(1→4)- 2-déoxy-2-sulfoamino-6-*O*-sulfo- α-D-glucopyranosyl-(1→4)-acide 2-*O*-sulfo- α-L-idopyranosyluronique-(1→4)-2-déoxy-2-sulfoamino-6-*O*-sulfo-D-glucitol, sel de dodécasodium); (acide 4-déoxy-2-*O*-sulfo- α-L-thréo-hex-4-enopyranosyluronique - (1→4)- 2-déoxy-6-*O*-sulfo-2-sulfoamino- α -D-glucopyranosyl-(1→4)- acide 2-*O*-sulfo- α-L-idopyranosyluronique -(1→4)- 2-déoxy6-*O*-sulfo-2-sulfoamino- α-D-glucopyranosyl-(1→4)-acide 2-*O*-sulfo- α-L-idopyranosyluronique-(1→4)-2-déoxy-6-*O*-sulfo-2-sulfoamino-D-manitol, sel de dodécasodium) : Spectre proton dans D₂O, 600MHz, T=305K, δ en ppm: 3,25 (2H, m, H-2^{(III) et (V)}), 3,38 (1H, m, H-2^{(I)}), 3,61 (2H, t, J=10Hz, H-3^{(III) et (V)}), entre 3,70 et 3,83 (4H, massif, 2H-1^{(I)} et H-4^{(III) et (V)}), 3,86 (1H, t, J=5Hz, H-3^{(I)}), 4,00 (2H, m, H-5^{(III)et (V)}), 4,07 (1H, m, H-4^{(IV)}), 4,08 (1H, m, H-4^{(I)}), entre 4,10 et 4,45 (13H, massif, H-5^{(I)}/2H-6^{(I)}, H-2^{(II)}/H-3^{(II)}/H-4^{(II)}, 2H-6^{(III)}, H-2^{(IV)}/H-3^{(IV)}, 2H-6^{(V)}, H-3^{(VI)}), 4,60 (1H, s, H-2^{(VI)}), 4,62 (1H, s, H-5^{(II)}), 4,78 (1H, s, H-5^{(IV)}), 5,17 (1H, s, H-1^{(IV)}), 5,28 (1H, d, J=4Hz, H-1^{(II)}), 5,38 (1H, d, J=3Hz, H-1^{(V)}), 5,44 (1H, d, J=3Hz, H-1^{(III)}), 5,47 (1H, d, J=2Hz, H-1^{(VI)}), 5,96 (1H, d, J=5Hz, H-4^{(VI)})].

### EXEMPLE 4

Dans un réacteur on introduit une solution à 25°C de 100 mg d'un oligosaccharide de formule (II) dans laquelle n est égal à 3, R₁, R₂, R₃, R₅, R₇ et R₈ représentent un radical SO₃M, R₄ et R₆ représentent un atome d'hydrogène et M est sodium, dans 2 ml d'eau. Sous agitation, on ajoute en deux fois 30 mg de borohydrure de sodium. Le pH est alors ajusté entre 9 et 10 par addition d'une solution d'hydroxyde de sodium à 0,5 mol/l. Après 12 heures, on ajoute de façon progressive de l'acide acétique jusqu'à obtention d'un pH compris entre 4 et 5. Le mélange est agité 1 heure puis on réajuste le pH à 6,7 par addition de soude à 0,5 mol/l. Le mélange est alors dilué avec de l'eau distillée en quantité suffisante pour obtenir 20 ml. On ajoute 2 g d'acétate de sodium et on coule 3 volumes de méthanol. La suspension est filtrée sur membrane Whatman GF/B et le solide récupéré est rincé par 2 fois 1 ml de méthanol. Après séchage, on obtient 68 mg d'un solide blanc. Après contrôle CLHP (Chromatographie Liquide Haute Pression), le produit n'étant pas totalement réduit, on renouvelle de façon complète les toutes opérations précédentes. Après séchage, on obtient 45 mg d'un oligosaccharide de formule (I) pour lequel n est égal à 3, R₁, R₂, R₃, R₅, R₇ et R₈ représentent un radical SO₃M, R₄ et R₆ représentent un atome d'hydrogène et M est sodium, sous forme d'un mélange des diastéréoisomères. On note de I à VIII les sucres constitutifs des octasaccharides, I étant le résidu réduit et VIII le résidu acide uronique insaturé, [(acide 4-déoxy-2-*O*-sulfo- α-L-thréo-hex-4-enopyranosyluronique -(1→4)- 2-déoxy-6-*O*-sulfo-2-sulfoamino-α-D-glucopyranosyl-(1→4)- acide 2-*O*-sulfo- α-L-idopyranosyluronique -(1→4)- 2-déoxy-2-sulfoamino-6-*O*-sulfo- α-D-glucopyranosyl-(1→4)- acide 2-*O*-sulfo-α-L-idopyranosyluronique -(1→4)- 2-déoxy6-*O*-sulfo-2-sulfoamino- α-D-glucopyranosyl-(1→+4)- acide 2-*O*-sulfo- α-L-idopyranosyluronique-(1→4)-2-déoxy-6-*O*-sulfo-2-sulfoamino-D-glucitol, sel d'hexadécasodium) ; (acide 4-déoxy-2-*O*-sulfo- α-L-thréo-hex-4-enopyranosyluronique -(1→4)- 2-déoxy-6-*O*-sulfo-2-sulfoamino- α-D-glucopyranosyl-( 1→4)- acide 2-*O*-sulfo- α-L-idopyranosyluronique -( 1→4)- 2-déoxy-6-*O*-sulfo-2-sulfoamino- α-D-glucopyranosyl-(1→4)- acide 2-*O*-sulfo- α-L-idopyranosyluronique -(1→4)- 2-déoxy-6-*O*-sulfo-2-sulfoamino- α-D-glucopyranosyl-(1→4)- acide 2-*O*-sulfo- α-L-idopyranosyluronique-(1→4)-2-déoxy-6-*O*-sulfo-2-sulfoamino-D-manitol, sel d'hexadécasodium) : Spectre proton dans D₂O, 600MHz, T=305K, δ en ppm: 3,25 (3H, m, H-2^{(III)}, H-2^{(V)}, H-2^{(VII)}), 3,38 (1H, m, H-2^{(I)}), 3,61 (3H, m, H-3^{(III)}, H-3^{(V)}, H-3^{(VII)}), entre 3,70 et 3,83 (5H, massif, 2H-1^{(I)} et H-4^{(III)}, H-4^{(V)}, H-4^{(VII)}), 3,86 (1H, t, J=5Hz, H-3^{(I)}), 4,00 (3H, m, H-55^{(III)}, H-5^{(V)}, H-5^{(VII)}, 4,08 (3H, m, H-4^{(I)}, H-4^{(IV)}, H-4^{(VI)}, entre 4,10 et 4,45 (17H, massif, H-5^{(I)}/2H-6^{(I)}, H-2^{(II)}/H-3^{(II)}/H-4^{(II)}, 2H-6^{(III)}, H-2^{(IV)}/H-3^{(IV)}, 2H-6^{(V)}, H-2^{(VI)}/H-3^{(VI)}, 2H-6^{(VII)}, H-3^{(VIII)}), 4,59 (1H, s, H-2^{(VIII)}), 4,62 (1H, s, H-5^{(II)}), 4,78 (2H, s, H-5^{(IV)}, H-5^{(VI)}), 5,17 (2H, s, H-1^{(IV)}, H-1^{(VI)}), 5,28 (1H, d, J=4Hz, H-1^{(II)}), 5,38 (2H, m, H-1^{(V)}, H-1^{(VII)}, 5,44 (1H, d, J=3Hz, H-1^{(III)}), 5,47 (1H, s, H-1^{(VIII)}), 5,96 (1H, d, J=5Hz, H-4^{(VIII)})].

### EXEMPLE 5

Dans un réacteur on introduit une solution à 25°C de 65 mg d'un oligosaccharide de formule (II) dans laquelle n est égal à 4, R₁, R₂, R₃, R₅, R₇ et R₈ représentent un radical SO₃M, R₄ et R₆ représentent un atome d'hydrogène et M est sodium, dans 1,2 ml d'eau. Sous agitation, on ajoute en une fois 18 mg de borohydrure de sodium. Le pH est alors ajusté entre 9 et 10 par addition d'une solution d'hydroxyde de sodium à 0,5 mol/l. Après 12 heures, on ajoute de façon progressive de l'acide acétique jusqu'à obtention d'un pH compris entre 4 et 5. Le mélange est agité 1 heure puis on réajuste le pH à 6,7 par addition de soude à 0,5 mol/l. Le mélange est alors dilué avec 3 ml d'une solution aqueuse d'acétate de sodium à 10% et on coule 3 volumes de méthanol (12 ml). La suspension est filtrée et le solide récupéré est rincé par 3 ml de méthanol. Après séchage, on obtient 54 mg d'un oligosaccharide de formule (I) pour lequel n est égal à 4, R₁, R₂, R₃, R₅, R₇ et R₈ représentent un radical SO₃M, R₄ et R₆ représentent un atome d'hydrogène et M est sodium, sous forme d'un mélange des diastéréoisomères. On note de I à X les sucres constitutifs des décasaccharides, I étant le résidu réduit et X le résidu acide uronique insaturé. [(acide 4-déoxy-2-*O*-sulfo- α-L-thréo-hex-4-enopyranosyluronique -(1→4)- 2-déoxy-2-sulfoamino-6-*O*-sulfo- α-D-glucopyranosyl-(1→4)- acide 2-*O*-sulfo- α-L-idopyranosyluronique -(1→4)- 2-déoxy-6-*O*-sulfo-2-sulfoamino- α-D-glucopyranosyl-(1→4)- acide 2-*O*-sulfo- α-L-idopyranosyluronique -(1→4)- 2-déoxy-6-*O*-sulfo-2-sulfoamino- α-D-glucopyranosyl-(1→4)- acide 2-*O*-sulfo-α-L-idopyranosyluronique -(1→4)- 2-déoxy6-*O*-sulfo-2-sulfoamino- α-D-glucopyranosyl-(1→4)- acide 2-*O*-sulfo- α-L-idopyranosyluronique-( 1→4)-2-déoxy-6-*O*-sulfo-2-sulfoamino-D-glucitol, sel d'eicosodium) ; (acide 4-déoxy-2-*O*-sulfo-α-L-thréo-hex-4-enopyranosyluronique-(1→4)- 2-déoxy-6-*O*-sulfo-2-sulfoamino-α-D-glucopyranosyl-(1→4)- acide 2-*O*-sulfo- α-L-idopyranosyluronique -(1→4)- 2-déoxy-6-*O*-sulfo-2-sulfoamino- α-D-glucopyranosyl-(1 →4)- acide 2-*O*-sulfo- α-L-idopyranosyluronique -(1 →4)- 2-déoxy-6-*O*-sulfo-2-sulfoamino- α-D-glucopyranosyl-(1→4)- acide 2-*O*-sulfo- α-L-idopyranosyluronique -(1→4)- 2-déoxy-6-*O*-sulfo-2-sulfoamino- α-D-glucopyranosyl-( 1→4)- acide 2-*O*-sulfo- α-L-idopyranosyluronique-(1→4)-2-déoxy-6-*O*-sulfo-2-sulfoamino-D-manitol, sel d'eicosodium) : Spectre proton dans D₂O, 600MHz, T=303K, δ en ppm: 3,23 (4H, m, H-2^{(III)}, H-2^{(V)}, H-2^{(VII)}, H-2^{(IX)}), 3,35 (1H, m, H2^{(I)}), 3,59 (4H, m, H-3^{(III)}, H-3^{(V)}, H-3^{(VII)}, H-3^{(IX)}), entre 3,65 et 3,80 (6H, m), 3,85 (1H, m, H-3^{(I)}), entre 3,90 et 4,40 (29H, m), 4,57 (1H, m, H-2^{(X)}), 4,59 (1H, m, H-5^{(II)}), 4,75 (3H, m, H-5^{(IV)}, H-5^{(VI)}, H-5^{(VIII)}), 5,15 (3H, m, H-1^{(IV)}, H-1^{(VI)}, H-1^{(VIII)}), 5,25 (1H, m, H-1^{(II)}), 5,37 (3H, m, H-1^{(V)}, H-1^{(VII)}, H-1^{(IX)}), 5,42 (1H, m, H-1^{(III)}), 5,45 (1H, m, H-1^{(X)}), 5,93 (1H, d, J=5Hz, H-4^{(X)}).

Les médicaments selon l'invention comprennent en tant que principe actif au moins un oligosaccharide de formule (I) ou un mélange d'oligosaccharides de formule (I), sous forme d'une composition dans laquelle il est associé à tout autre produit pharmaceutiquement compatible, pouvant être inerte ou physiologiquement actif. Les médicaments selon l'invention peuvent être employés par voie intraveineuse, sous-cutanée, orale, rectale, topique ou pulmonaire (inhalation).

Les compositions stériles pour administration intraveineuse ou sous-cutanée, sont généralement des solutions aqueuses. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation. Elles peuvent également être préparées sous forme de compositions solides stériles qui peuvent être dissoutes au moment de l'emploi dans de l'eau stérile ou tout autre milieu stérile injectable.

Comme compositions solides pour administration orale, peuvent être utilisés des comprimés, des pilules, des poudres (capsules de gélatine, cachets) ou des granulés. Dans ces compositions, le principe actif est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un agent favorisant l'absorption orale, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 0,5 mg et 10 mg par kg par jour par voie sous-cutanée soit 3 à 60 mg par jour pour un adulte de 60 kg.

D'une façon générale, le médecin déterminera la posologie appropriée en fonction de l'âge, du poids et de tous les autres facteurs propres au sujet à traiter.

L'invention concerne également l'utilisation des oligosaccharides selon l'invention pour la préparation d'un médicament utile pour la prévention ou le traitement de maladies liées à un processus inflammatoire impliquant la production de nitrite oxyde (NO) ou utile pour la survie et la croissance des motoneurones.

L'invention est particulièrement avantageuse pour l'utilisation des oligosaccharides de formule (I) pour la préparation de médicaments utiles pour la prévention et le traitement des ischémies cérébrales, cardiaques ou vasculaires périphériques, d'ostéoarthrites, des traumatismes du système nerveux central, des traumatismes crâniens, spinaux et cranio-spinaux, de la sclérose en plaques, des douleurs neuropathiques et des neuropathies périphériques, des maladies du motoneurone, de la sclérose latérale amyotrophique, du neuro-sida, de la maladie d'Alzheimer, de la maladie de Parkinson et de la Chorée de Huntington.

L'invention concerne également la méthode de prévention et/ou de traitement de maladies liées à un processus inflammatoire impliquant la production de substances cytotoxiques telle que le nitrite oxyde (NO) et des maladies liées à la survie et la croissance des motoneurones. Les oligosaccharides de formule (I) peuvent ainsi être utilisées pour la prévention et/ou le traitement des maladies neurodégénératives pour lesquelles l'inflammation cérébrale joue un rôle délétère pouvant conduire à la mort parmi lesquelles on peut citer les ischémies cérébrales, les ischémies cardiaques (infarctus du myocarde), les ischémies périphériques, les traumatismes du système nerveux central et notamment les traumatismes crâniens, spinaux et cranio-spinaux, la sclérose en plaques, les douleurs neuropathiques et les neuropathies périphériques, les maladies du motoneurone comme la sclérose latérale amyotrophique, l'atrophie spinale progressive, l'atrophie musculaire infantile et la sclérose latérale primaire, le neuro-sida, la maladie d'Alzheimer, la maladie de Parkinson et la Chorée de Huntington et certaines formes d'ostéoarthrites notamment à localisation articulaire.

La présente invention concerne aussi la méthode de prévention et/ou de traitement des maladies motoneuronales telles que la sclérose latérale amyotrophique, l'atrophie spinale progressive, l'atrophie musculaire infantile et la sclérose latérale primaire,

## Revendications

1. Composition pharmaceutique contenant en tant que principe actif un oligosaccharide de formule : 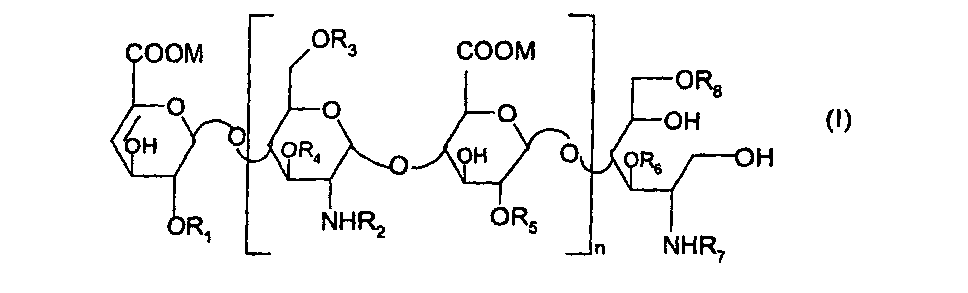 dans laquelle n est un entier de 0 à 25, R₁, R₃, R₄, R₅, R₆ et R₈, identiques ou différents, représentent un atome d'hydrogène ou un radical SO₃M, R₂ et R₇, identiques ou différents, représentent un atome d'hydrogène ou un radical SO₃M ou COCH₃ et M est sodium, calcium, magnésium ou potassium ou un mélange de ces oligosaccharides à l'exception de ceux pour lesquels n est égal à 0 et soit R₁, R₆ et R₈ sont des atomes d'hydrogène, R₇ représente un radical SO₃M ou COCH₃ et M est sodium, soit R₁ et R₆ représentent un atome d'hydrogène, R₇ représente un radical COCH₃, R₈ représente un radical SO₃M et M est sodium, soit R₆ représente un hydrogène, R₁, R₇ et R₈ représentent un radical SO₃M et M est sodium, soit R₆ et R₇ représentent des atomes d'hydrogène, R₁ et R₈ représentent un radical SO₃M et M est sodium, soit R₁, R₆ et R₇ représentent un atome d'hydrogène, R₈ représente un radical SO₃M et M est sodium.

2. Composition pharmaceutique selon la revendication 1 contenant un oligosaccharide de formule (I) pour lequel R₄ et R₆ représentent un atome d'hydrogène ou un mélange de ces oligosaccharides.

3. Composition pharmaceutique selon l'une des revendications 1 ou 2 contenant un oligosaccharide de formule (I) pour lequel n est un entier de 0 à 10, un mélange de ces oligosaccharides.

4. Composition pharmaceutique selon l'une des revendications 1 ou 2 contenant un oligosaccharide de formule (I) pour lequel n est un entier de 0 à 6, un mélange de ces oligosaccharides.

5. Composition pharmaceutique selon l'une des revendications 1 ou 2 contenant un oligosaccharide de formule (I) pour lequel n est un entier de 1 à 6, un mélange de ces oligosaccharides.

6. Composition pharmaceutique selon la revendication 1 contenant un oligosaccharide de formule (I) pour lequel n est égal à 1, R₁, R₂, R₃, R₅, R₇ et R₈, représentent un radical SO₃M, R₄ et R₆ représentent un atome d'hydrogène et M est sodium.

7. Composition pharmaceutique selon la revendication 1 contenant un oligosaccharide de formule (I) pour lequel n est égal à 2, R₁, R₂, R₃, R₅, R₇ et R₈, représentent un radical SO₃M, R₄ et R₆ représentent un atome d'hydrogène et M est sodium.

8. Composition pharmaceutique selon la revendication 1 contenant un oligosaccharide de formule (I) pour lequel n est égal à 3, R₁, R₂, R₃, R₅, R₇ et R₈, représentent un radical SO₃M, R₄ et R₆ représentent un atome d'hydrogène et M est sodium.

9. Composition pharmaceutique selon la revendication 1 contenant un oligosaccharide de formule (I) pour lequel n est égal à 4, R₁, R₂, R₃, R₅, R₇ et R₈, représentent un radical SO₃M, R₄ et R₆ représentent un atome d'hydrogène et M est sodium.

10. Oligosaccharide de formule : 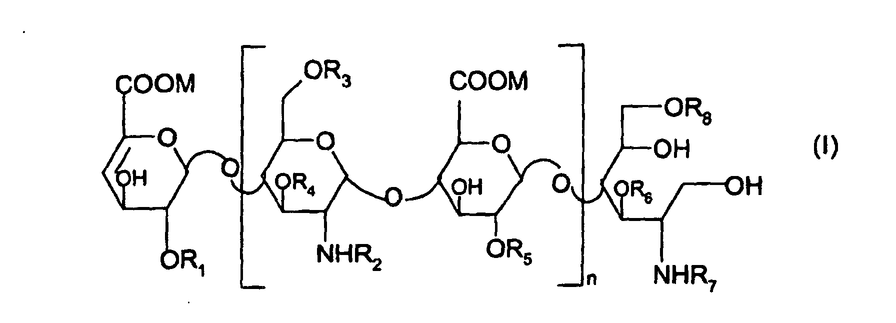 dans laquelle n est un entier de 0 à 25, R₁, R₃, R₄, R₅, R₆ et R₈, identiques ou différents, représentent un atome d'hydrogène ou un radical SO₃M, R₂ et R₇, identiques ou différents, représentent un atome d'hydrogène ou un radical SO₃M ou COCH₃ et M est sodium, calcium, magnésium ou potassium, à l'exception de ceux pour lesquels n est égal à 0 et soit R₁, R₆ et R₈ sont des atomes d'hydrogène, R₇ représente un radical SO₃M ou COCH₃ et M est sodium, soit R₁ et R₆ représentent un atome d'hydrogène, R₇ représente un radical COCH₃, R₈ représente un radical SO₃M et M est sodium, soit R₆ représente un hydrogène, R₁, R₇ et R₈ représentent un radical SO₃M et M est sodium, soit R₆ et R₇ représentent des atomes d'hydrogène, R₁ et R₈ représentent un radical SO₃M et M est sodium, soit R₁, R₆ et R₇ représentent un atome d'hydrogène, R₈ représente un radical SO₃M et M est sodium.

11. Oligosaccharide de formule (I) selon la revendication 10 pour lequel R₄ et R₆ représentent un atome d'hydrogène.

12. Oligosaccharide de formule (I) selon l'une des revendications 10 ou 11 pour lequel n est un entier de 0 à 10.

13. Oligosaccharide de formule (I) selon l'une des revendications 10 ou 11 pour lequel n est un entier de 0 à 6.

14. Oligosaccharide de formule (I) selon l'une des revendications 10 ou 11 pour lequel n est un entier de 1 à 6.

15. Oligosaccharide de formule (I) selon la revendication 10 pour lequel n est égal à 1, R₁, R₂, R₃, R₅, R₇ et R₈, représentent un radical SO₃M, R₄ et R₆ représentent un atome d'hydrogène et M est sodium.

16. Oligosaccharide de formule (I) selon la revendication 10 pour lequel n est égal à 2, R₁, R₂, R₃, R₅, R₇ et R₈, représentent un radical SO₃M, R₄ et R₆ représentent un atome d'hydrogène et M est sodium.

17. Oligosaccharide de formule (I) selon la revendication 10 pour lequel n est égal à 3, R₁, R₂, R₃, R₅, R₇ et R₈, représentent un radical SO₃M, R₄ et R₆ représentent un atome d'hydrogène et M est sodium.

18. Oligosaccharide de formule (I) selon la revendication 10 pour lequel n est égal à 4, R₁, R₂, R₃, R₅, R₇ et R₈, représentent un radical SO₃M, R₄ et R₆ représentent un atome d'hydrogène et M est sodium.

19. Procédé de préparation des oligosaccharides de formule (I) selon la revendication 10 **caractérisé en ce que** l'on fait réagir un borohydrure de métal alcalin ou d'ammonium quaternaire sur des oligosaccharides de formule : 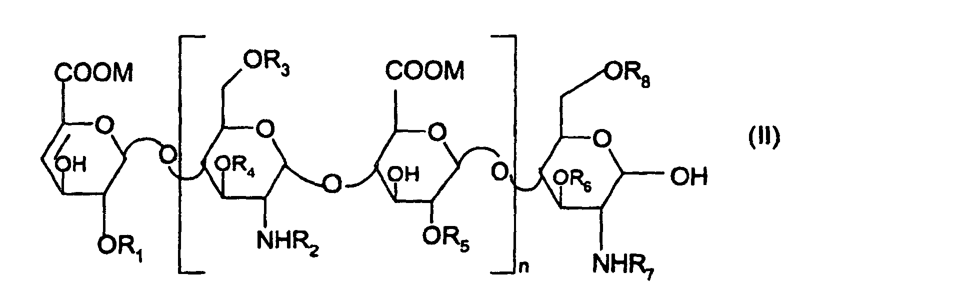 dans laquelle n est un entier de 0 à 25, R₁, R₃, R₄ et R₅, identiques ou différents, représentent un atome d'hydrogène ou un radical SO₃M, R₂ et R₆, identiques ou différents, représentent un atome d'hydrogène ou un radical SO₃M ou COCH₃ et M est sodium, calcium, magnésium ou potassium et isole les oligosaccharides.

20. Procédé selon la revendication 19 **caractérisé en ce que** l'on effectue la réaction en milieu aqueux, à une température voisine de 25°C et à un pH de 7 à 10.

21. Procédé selon l'une des revendications 19 et 20 **caractérisé en ce que** le pH réactionnel est de 9 à 10.

22. Procédé selon des revendications 19 à 21 **caractérisé en ce que** le borohydrure de métal alcalin ou d'ammonium quaternaire est le borohydrure de lithium, sodium, de potassium ou de tétrabutylammonium.

23. Utilisation des oligosaccharides de formule : 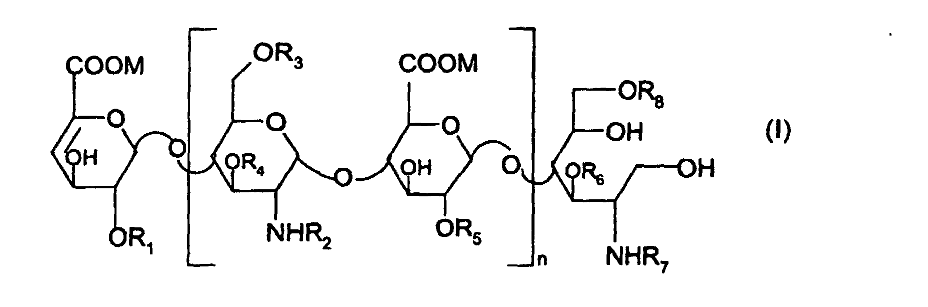 dans laquelle n est un entier de 0 à 25, R₁, R₃, R₄, R₅, R₆ et R₈, identiques ou différents, représentent un atome d'hydrogène ou un radical SO₃M, R₂ et R₇, identiques ou différents, représentent un atome d'hydrogène ou un radical SO₃M ou COCH₃ et M est sodium, calcium, magnésium ou potassium ou un mélange de ces oligosaccharides pour la préparation d'un médicament utile pour la prévention ou le traitement de maladies liées à un processus inflammatoire impliquant la production de nitrite oxyde (NO).

24. Utilisation des oligosaccharides de formule (I) selon la revendication 23 pour la préparation de médicaments utiles pour la prévention et le traitement des ischémies cérébrales, cardiaques ou vasculaires périphériques, d'ostéoarthrites, des traumatismes du système nerveux central, des traumatismes crâniens, spinaux et cranio-spinaux, de la sclérose en plaques, des douleurs neuropathiques et des neuropathies périphériques, des maladies du motoneurone, de la sclérose latérale amyotrophique, du neuro-sida, de la maladie d'Alzheimer, de la maladie de Parkinson et de la Chorée de Huntington.

25. Utilisation des oligosaccharides de formule (I) 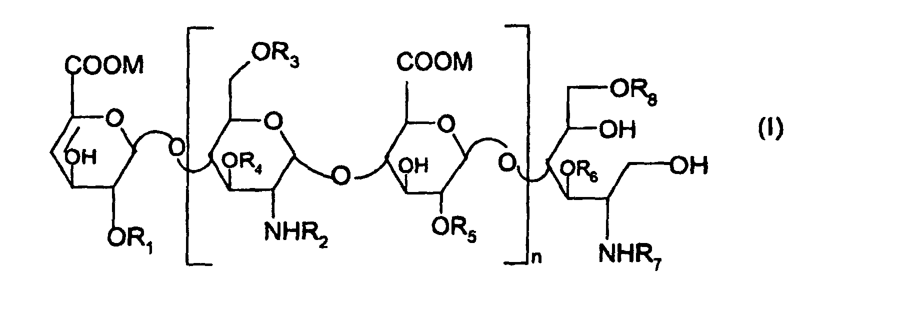 dans laquelle n est un entier de 0 à 25, R₁, R₃, R₄, R₅, R₆ et R₈, identiques ou différents, représentent un atome d'hydrogène ou un radical SO₃M, R₂ et R₇, identiques ou différents, représentent un atome d'hydrogène ou un radical SO₃M ou COCH₃ et M est sodium, calcium, magnésium ou potassium ou un mélange de ces oligosaccharides pour la préparation d'un médicament pour la prévention et/ou le traitement des maladies liées à la survie et la croissance des motoneurones.

## Claims

1. Pharmaceutical composition containing, as active principle, an oligosaccharide of formula: 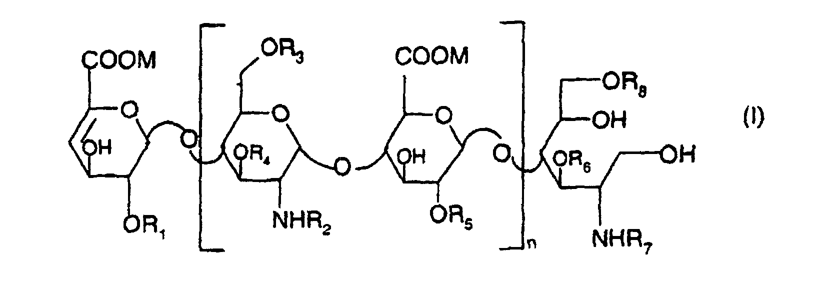 in which n is an integer from 0 to 25, R₁, R₃, R₄, R₅, R₆ and R₈, which are identical or different, represent a hydrogen atom or an SO₃M radical, R₂ and R₇, which are identical or different, represent a hydrogen atom or an SO₃M or COCH₃ radical, and M is sodium, calcium, magnesium or potassium, or a mixture of these oligosaccharides, with the exception of those for which n is equal to 0 and either R₁, R₆ and R₈ are hydrogen atoms, R₇ represents an SO₃M or COCH₃ radical and M is sodium, or R₁ and R₆ represent a hydrogen atom, R₇ represents a COCH₃ radical, R₈ represents an SO₃M radical and M is sodium, or R₆ represents a hydrogen, R₁, R₇ and R₈ represent an SO₃M radical and M is sodium, or R₆ and R₇ represent hydrogen atoms, R₁ and R₈ represent an SO₃M radical and M is sodium, or R₁, R₆ and R₇ represent a hydrogen atom, R₈ represents an SO₃M radical and M is sodium.

2. Pharmaceutical composition according to Claim 1, containing an oligosaccharide of formula (I) for which R₄ and R₆ represent a hydrogen atom, or a mixture of these oligosaccharides.

3. Pharmaceutical composition according to either of Claims 1 and 2, containing an oligosaccharide of formula (I) for which n is an integer from 0 to 10, a mixture of these oligosaccharides.

4. Pharmaceutical composition according to either of Claims 1 and 2, containing an oligosaccharide of formula (I) for which n is an integer from 0 to 6, a mixture of these oligosaccharides.

5. Pharmaceutical composition according to either of Claims 1 and 2, containing an oligosaccharide of formula (I) for which n is an integer from 1 to 6, a mixture of these oligosaccharides.

6. Pharmaceutical composition according to Claim 1, containing an oligosaccharide of formula (I) for which n is equal to 1, R₁, R₂, R₃, R₅, R₇ and R₈ represent an SO₃M radical, R₄ and R₆ represent a hydrogen atom and M is sodium.

7. Pharmaceutical composition according to Claim 1, containing an oligosaccharide of formula (I) for which n is equal to 2, R₁, R₂, R₃, R₅, R₇ and R₈ represent an SO₃M radical, R₄ and R₆ represent a hydrogen atom and M is sodium.

8. Pharmaceutical composition according to Claim 1, containing an oligosaccharide of formula (I) for which n is equal to 3, R₁, R₂, R₃, R₅, R₇ and R₈ represent an SO₃M radical, R₄ and R₆ represent a hydrogen atom and M is sodium.

9. Pharmaceutical composition according to Claim 1, containing an oligosaccharide of formula (I) for which n is equal to 4, R₁, R₂, R₃, R₅, R₇ and R₈ represent an SO₃M radical, R₄ and R₆ represent a hydrogen atom and M is sodium.

10. Oligosaccharide of formula: 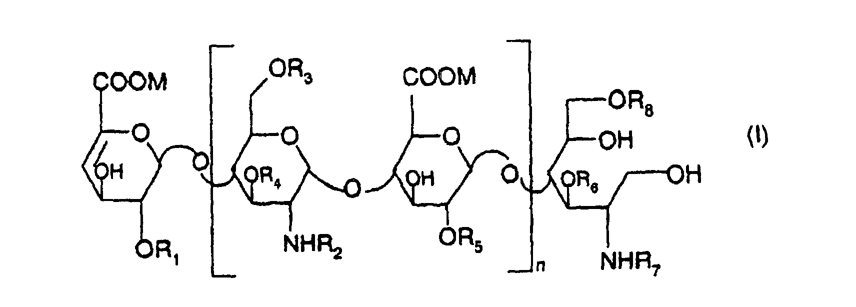 in which n is an integer from 0 to 25, R₁, R₃, R₄, R₅, R₆ and R₈, which are identical or different, represent a hydrogen atom or an SO₃M radical, R₂ and R₇, which are identical or different, represent a hydrogen atom or an SO₃M or COCH₃ radical, and M is sodium, calcium, magnesium or potassium, with the exception of those for which n is equal to 0 and either R₁, R₆ and R₈ are hydrogen atoms, R₇ represents an SO₃M or COCH₃ radical and M is sodium, or R₁ and R₆ represent a hydrogen atom, R₇ represents a COCH₃ radical, R₈ represents an SO₃M radical and M is sodium, or R₆ represents a hydrogen, R₁, R₇ and R₈ represent an SO₃M radical and M is sodium, or R₆ and R₇ represent hydrogen atoms, R₁ and R₈ represent an SO₃M radical and M is sodium, or R₁, R₆ and R₇ represent a hydrogen atom, R₈ represents an SO₃M radical and M is sodium.

11. Oligosaccharide of formula (I) according to Claim 10, for which R₄ and R₆ represent a hydrogen atom.

12. Oligosaccharide of formula (I) according to either of Claims 10 and 11, for which n is an integer from 0 to 10.

13. Oligosaccharide of formula (I) according to either of Claims 10 and 11, for which n is an integer from 0 to 6.

14. Oligosaccharide of formula (I) according to either of Claims 10 and 11, for which n is an integer from 1 to 6.

15. Oligosaccharide of formula (I) according to Claim 10, for which n is equal to 1, R₁, R₂, R₃, R₅, R₇ and R₈ represent an SO₃M radical, R₄ and R₆ represent a hydrogen atom and M is sodium.

16. Oligosaccharide of formula (I) according to Claim 10, for which n is equal to 2, R₁, R₂, R₃, R₅, R₇ and R₈ represent an SO₃M radical, R₄ and R₆ represent a hydrogen atom and M is sodium.

17. Oligosaccharide of formula (I) according to Claim 10, for which n is equal to 3, R₁, R₂, R₃, R₅, R₇ and R₈ represent an SO₃M radical, R₄ and R₆ represent a hydrogen atom and M is sodium.

18. Oligosaccharide of formula (I) according to Claim 10, for which n is equal to 4, R₁, R₂, R₃, R₅, R₇ and R₈ represent an SO₃M radical, R₄ and R₆ represent a hydrogen atom and M is sodium.

19. Method for preparing the oligosaccharides of formula (I) according to Claim 10, **characterized in that** an alkali metal borohydride or a quaternary ammonium borohydride is reacted with oligosaccharides of formula: 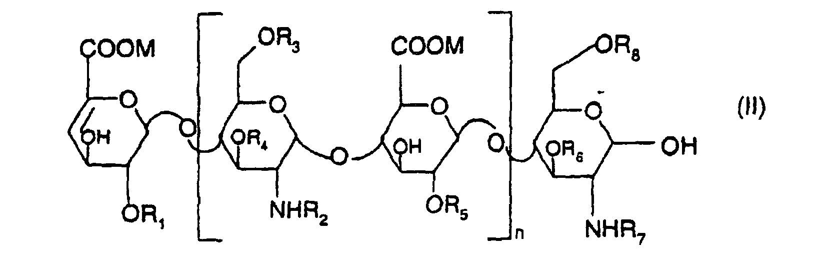 in which n is an integer from 0 to 25, R₁, R₃, R₄ and R₅, which are identical or different, represent a hydrogen atom or an SO₃M radical, R₂ and R₆, which are identical or different, represent a hydrogen atom or an SO₃M or COCH₃ radical, and M is sodium, calcium, magnesium or potassium, and the oligosaccharides are isolated.

20. Method according to Claim 19, **characterized in that** the reaction is carried out in aqueous medium, at a temperature in the vicinity of 25°C and at a pH from 7 to 10.

21. Method according to either of Claims 19 and 20, **characterized in that** the reaction pH is from 9 to 10.

22. Method according to Claims 19 to 21, **characterized in that** the alkali metal borohydride or quaternary ammonium borohydride is lithium borohydride, sodium borohydride, potassium borohydride or tetrabutylammonium borohydride.

23. Use of the oligosaccharides of formula: 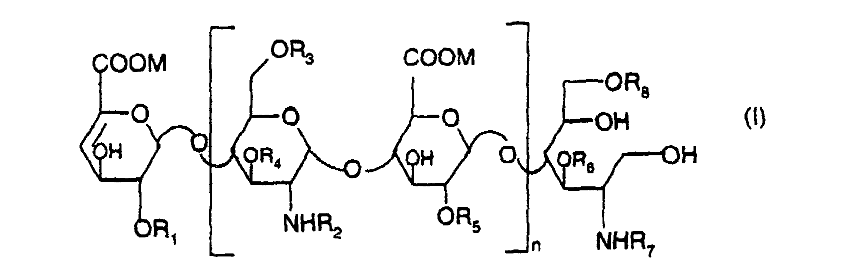 in which n is an integer from 0 to 25, R₁, R₃, R₄, R₅, R₆ and R₈, which are identical or different, represent a hydrogen atom or an SO₃M radical, R₂ and R₇, which are identical or different, represent a hydrogen atom or an SO₃M or COCH₃ radical, and M is sodium, calcium, magnesium or potassium, or a mixture of these oligosaccharides, for preparing a medicinal product which is useful for preventing or treating diseases associated with an inflammatory process involving the production of nitrite oxide (NO).

24. Use of the oligosaccharides of formula (I) according to Claim 23, for preparing medicinal products which are useful for preventing and treating cerebral, cardiac or peripheral vascular ischemias, osteoarthritis, traumas of the central nervous system, cranial traumas, spinal and craniospinal traumas, multiple sclerosis, neuropathic pain and peripheral neuropathies, motoneuron diseases, amyotrophic lateral sclerosis, neuro-AIDS, Alzheimer's disease, Parkinson's disease and Huntington's chorea.

25. Use of the oligosaccharides of formula (I) : 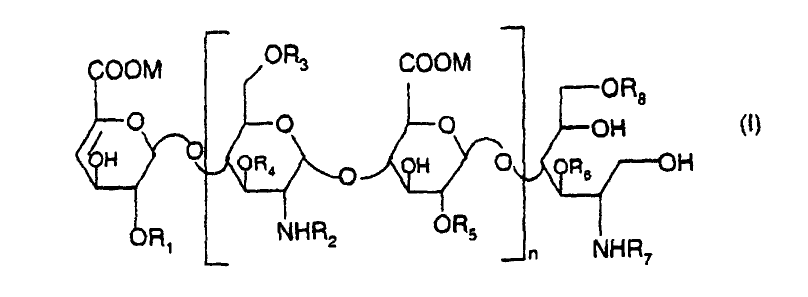 in which n is an integer from 0 to 25, R₁, R₃, R₄, R₅, R₆ and R₈, which are identical or different, represent a hydrogen atom or an SO₃M radical, R₂ and R₇, which are identical or different, represent a hydrogen atom or an SO₃M or COCH₃ radical, and M is sodium, calcium, magnesium or potassium, or a mixture of these oligosaccharides, for preparing a medicinal product for preventing and/or treating diseases associated with motoneuron survival and growth.

## Patentansprüche

1. Pharmazeutische Zusammensetzung, enthaltend als Wirkstoff ein Oligosaccharid der Formel: 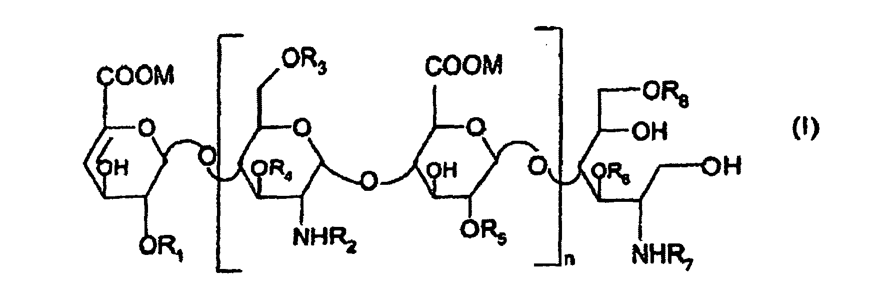 worin n eine ganze Zahl von 0 bis 25 ist, R₁, R₃, R₄, R₅, R₆ und R₈, die gleich oder verschieden sind, ein Wasserstoffatom oder einen SO₃M-Rest darstellen, R₂ und R₇, die gleich oder verschieden sind, ein Wasserstoffatom oder einen SO₃M- oder COCH₃-Rest darstellen und M Natrium, Calcium, Magnesium oder Kalium ist, oder ein Gemisch dieser Oligosaccharide mit Ausnahme derjenigen, für die n gleich 0 ist und entweder R₁, R₆ und R₈ Wasserstoffatome sind, R₇ einen SO₃M- oder COCH₃-Rest darstellt und M Natrium ist, oder R₁ und R₆ ein Wasserstoffatom darstellen, R₇ einen COCH₃-Rest darstellt, R₈ einen SO₃M-Rest darstellt und M Natrium ist, oder R₆ einen Wasserstoff darstellt, R₁, R₇ und R₈ einen SO₃M-Rest darstellen und M Natrium ist, oder R₆ und R₇ Wasserstoffatome darstellen, R₁ und R₈ einen SO₃M-Rest darstellen und M Natrium ist, oder R₁, R₆ und R₇ ein Wasserstoffatom darstellen, R₈ einen SO₃M-Rest darstellt und M Natrium ist.

2. Pharmazeutische Zusammensetzung gemäß Anspruch 1, enthaltend ein Oligosaccharid der Formel (I), worin R₄ und R₆ ein Wasserstoffatom darstellen, oder ein Gemisch dieser Oligosaccharide.

3. Pharmazeutische Zusammensetzung gemäß einem der Ansprüche 1 oder 2, enthaltend ein Oligosaccharid der Formel (I), worin n eine ganze Zahl von 0 bis 10 ist, ein Gemisch dieser Oligosaccharide.

4. Pharmazeutische Zusammensetzung gemäß einem der Ansprüche 1 oder 2, enthaltend ein Oligosaccharid der Formel (I), worin n eine ganze Zahl von 0 bis 6 ist, ein Gemisch dieser Oligosaccharide.

5. Pharmazeutische Zusammensetzung gemäß einem der Ansprüche 1 oder 2, enthaltend ein Oligosaccharid der Formel (I), worin n eine ganze Zahl von 1 bis 6 ist, ein Gemisch dieser Oligosaccharide.

6. Pharmazeutische Zusammensetzung gemäß Anspruch 1, enthaltend ein Oligosaccharid der Formel (I), worin n gleich 1 ist, R₁, R₂, R₃, R₅, R₇ und R₈ einen SO₃M-Rest darstellen, R₄ und R₆ ein Wasserstoffatom darstellen und M Natrium ist.

7. Pharmazeutische Zusammensetzung gemäß Anspruch 1, enthaltend ein Oligosaccharid der Formel (I), worin n gleich 2 ist, R₁, R₂, R₃, R₅, R₇ und R₈ einen SO₃M-Rest darstellen, R₄ und R₆ ein Wasserstoffatom darstellen und M Natrium ist.

8. Pharmazeutische Zusammensetzung gemäß Anspruch 1, enthaltend ein Oligosaccharid der Formel (I), worin n gleich 3 ist, R₁, R₂, R₃, R₅, R₇ und R₈ einen SO₃M-Rest darstellen, R₄ und R₆ ein Wasserstoffatom darstellen und M Natrium ist.

9. Pharmazeutische Zusammensetzung gemäß Anspruch 1, enthaltend ein Oligosaccharid der Formel (I), worin n gleich 4 ist, R₁, R₂, R₃, R₅, R₇ und R₈ einen SO₃M-Rest darstellen, R₄ und R₆ ein Wasserstoffatom darstellen und M Natrium ist.

10. Oligosaccharid der Formel: 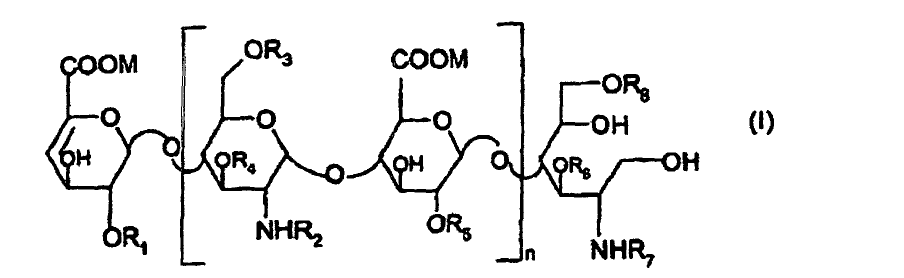 worin n eine ganze Zahl von 0 bis 25 ist, R₁, R₃, R₄, R₅, R₆ und R₈, die gleich oder verschieden sind, ein Wasserstoffatom oder einen SO₃M-Rest darstellen, R₂ und R₇, die gleich oder verschieden sind, ein Wasserstoffatom oder einen SO₃M- oder COCH₃-Rest darstellen und M Natrium, Calcium, Magnesium oder Kalium ist, mit Ausnahme derjenigen, für die n gleich 0 ist und entweder R₁, R₆ und R₈ Wasserstoffatome sind, R₇ einen SO₃M- oder COCH₃-Rest darstellt und M Natrium ist, oder R₁ und R₆ ein Wasserstoffatom darstellen, R₇ einen COCH₃-Rest darstellt, R₈ einen SO₃M-Rest darstellt und M Natrium ist, oder R₆ einen Wasserstoff darstellt, R₁, R₇ und R₈ einen SO₃M-Rest darstellen und M Natrium ist, oder R₆ und R₇ Wasserstoffatome darstellen, R₁ und R₈ einen SO₃M-Rest darstellen und M Natrium ist, oder R₁, R₈ und R₇ ein Wasserstoffatom darstellen, R₈ einen SO₃M-Rest darstellt und M Natrium ist.

11. Oligosaccharid der Formel (I) gemäß Anspruch 10, worin R₄ und R₆ ein Wasserstoffatom darstellen.

12. Oligosaccharid der Formel (I) gemäß einem der Ansprüche 10 oder 11, worin n eine ganze Zahl von 0 bis 10 ist.

13. Oligosaccharid der Formel (I) gemäß einem der Ansprüche 10 oder 11, worin n eine ganze Zahl von 0 bis 6 ist.

14. Oligosaccharid der Formel (I) gemäß einem der Ansprüche 10 oder 11, worin n eine ganze Zahl von 1 bis 6 ist.

15. Oligosaccharid der Formel (I) gemäß Anspruch 10, worin n gleich 1 ist, R₁, R₂, R₃, R₅, R₇ und R₈ einen SO₃M-Rest darstellen, R₄ und R₆ ein Wasserstoffatom darstellen und M Natrium ist.

16. Oligosaccharid der Formel (I) gemäß Anspruch 10, worin n gleich 2 ist, R₁, R₂, R₃, R₅, R₇ und R₈ einen SO₃M-Rest darstellen, R₄ und R₆ ein Wasserstoffatom darstellen und M Natrium ist.

17. Oligosaccharid der Formel (I) gemäß Anspruch 10, worin n gleich 3 ist, R₁, R₂, R₃, R₅, R₇ und R₈ einen SO₃M-Rest darstellen, R₄ und R₆ ein Wasserstoffatom darstellen und M Natrium ist.

18. Oligosaccharid der Formel (I) gemäß Anspruch 10, worin n gleich 4 ist, R₁, R₂, R₃, R₅, R₇ und R₈ einen SO₃M-Rest darstellen, R₄ und R₆ ein Wasserstoffatom darstellen und M Natrium ist.

19. Verfahren zur Herstellung der Oligosaccharide der Formel (I) gemäß Anspruch 10, **dadurch gekennzeichnet, dass** man ein Alkalimetall- oder quartäres Ammonium-borhydrid mit Oligosacchariden der Formel: 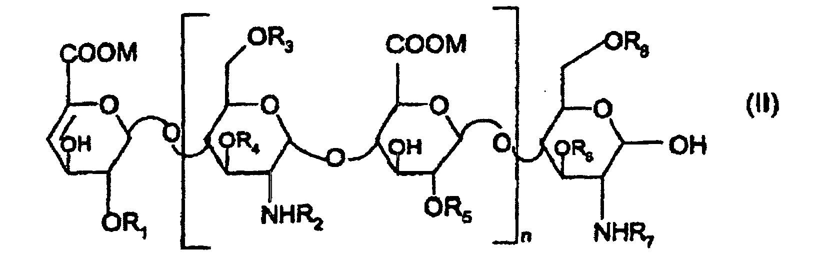 worin n eine ganze Zahl von 0 bis 25 ist, R₁, R₃, R₄ und R₅, die gleich oder verschieden sind, ein Wasserstoffatom oder einen SO₃M-Rest darstellen, R₂ und R₆, die gleich oder verschieden sind, ein Wasserstoffatom oder einen SO₃M- oder COCH₃-Rest darstellen und M Natrium, Calcium, Magnesium oder Kalium ist, umsetzt und die Oligosaccharide isoliert.

20. Verfahren gemäß Anspruch 19, **dadurch gekennzeichnet, dass** man die Reaktion in wässrigem Medium bei einer Temperatur nahe 25 °C und bei einem pH von 7 bis 10 ausführt.

21. Verfahren gemäß einem der Ansprüche 19 und 20, **dadurch gekennzeichnet, dass** der pH der Reaktion 9 bis 10 beträgt.

22. Verfahren gemäß Ansprüchen 19 bis 21, **dadurch gekennzeichnet, dass** das Alkalimetall- oder quartäre Ammonium-borhydrid Lithium-, Natrium-, Kalium- oder Tetrabutylammonium-borhydrid ist.

23. Verwendung der Oligosaccharide der Formel: 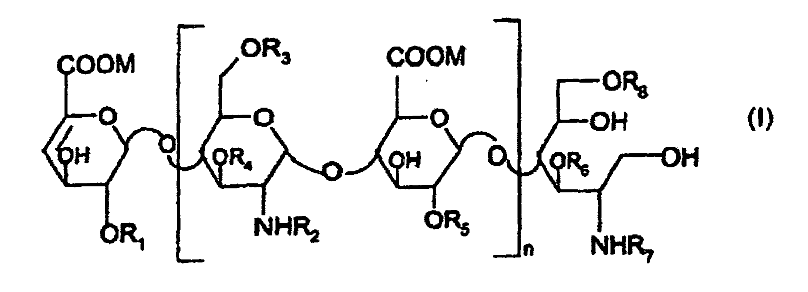 worin n eine ganze Zahl von 0 bis 25 ist, R₁, R₃, R₄, R₅, R₆ und R₈, die gleich oder verschieden sind, ein Wasserstoffatom oder einen SO₃M-Rest darstellen, R₂ und R₇, die gleich oder verschieden sind, ein Wasserstoffatom oder einen SO₃M- oder COCH₃-Rest darstellen und M Natrium, Calcium, Magnesium oder Kalium ist, oder eines Gemischs dieser Oligosaccharide für die Herstellung eines Medikaments, das sich für die Prophylaxe oder die Behandlung von Erkrankungen eignet, die mit einem entzündlichen Prozess, der die Produktion von Stickoxid (NO) einschließt, zusammenhängen.

24. Verwendung der Oligosaccharide der Formel (I) gemäß Anspruch 23 für die Herstellung von Medikamenten, die sich eignen für die Prophylaxe und die Behandlung der zerebralen, kardialen oder peripheren vaskulären Ischämien, von Osteoarthritiserkrankungen, der Traumen des zentralen Nervensystems, der Schädel-, spinalen oder cranio-spinalen Traumen, der Multiplen Sklerose, der neuropathischen Schmerzen und der peripheren Neuropathien, der Motoneuronerkrankungen, der amytrophen Lateralsklerose, von Neuro-AIDS, der Alzheimerkrankheit, der Parkinsonkrankheit und von Chorea Huntington.

25. Verwendung der Oligosaccharide der Formel (I) 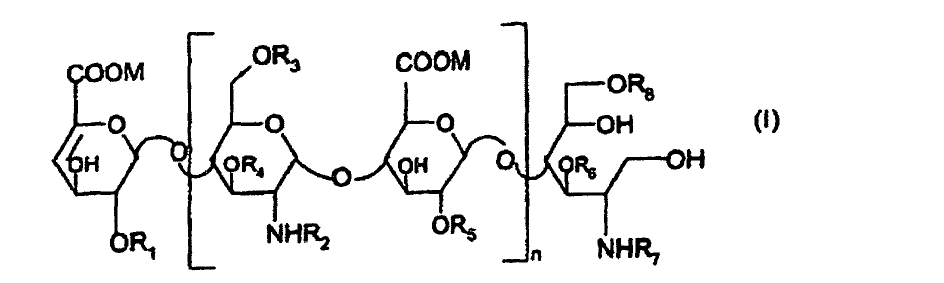 worin n eine ganze Zahl von 0 bis 25 ist, R₁, R₃, R₄, R₅, R₆ und R₈, die gleich oder verschieden sind, ein Wasserstoffatom oder einen SO₃M-Rest darstellen, R₂ und R₇, die gleich oder verschieden sind, ein Wasserstoffatom oder einen SO₃M- oder COCH₃-Rest darstellen und M Natrium, Calcium, Magnesium oder Kalium ist, oder eines Gemischs dieser Oligosaccharide für die Herstellung eines Medikaments für die Prophylaxe und/oder die Behandlung der Erkrankungen, die mit dem Überleben und dem Wachstum der Motoneuronen zusammenhängen.
